(19) Europäisches Patentamt / European Patent Office / Office européen des brevets

(11) **EP 3 718 797 B1**

(12) **EUROPEAN PATENT SPECIFICATION**

(45) Date of publication and mention
of the grant of the patent:
**17.06.2026 Bulletin 2026/25**

(21) Application number: **19167632.9**

(22) Date of filing: **05.04.2019**

(51) International Patent Classification (IPC):
**B60H 1/00** *(2006.01)*   **B61D 27/00** *(2006.01)*
**B64D 13/00** *(2006.01)*   **B60C 23/00** *(2006.01)*

(52) Cooperative Patent Classification (CPC):
**B64D 13/06; B60H 1/00371; B60H 1/00742;
B60H 1/00778; B60H 1/00978; B60H 1/04;
B61D 27/00; B61D 41/04; B64D 11/06;** Y02T 50/50

(54) **MASS TRANSPORTATION VEHICLE WITH PASSENGER DETECTION**

PERSONENTRANSPORTFAHRZEUG MIT INSASSENERFASSUNG

VÉHICULE DE TRANSPORTS EN COMMUN AVEC DETECTION DE PASSAGERS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**07.10.2020 Bulletin 2020/41**

(73) Proprietor: **Ford Global Technologies, LLC
Dearborn, MI 48126 (US)**

(72) Inventors:
• **Dienhart, Bernd**
  **50859 Köln (DE)**
• **Elson, John Craig**
  **Farmington Hills, MI 48334 (US)**
• **Scheer, Volker**
  **52159 Roetgen (DE)**
• **Schuermanns, Klaus**
  **50739 Köln (DE)**
• **Klein, Maximilian**
  **40215 Düsseldorf (DE)**
• **Maranville, Clay Wesley**
  **Ypsilanti, 48197 (US)**
• **Hirn, Thomas**
  **52062 Aachen (DE)**

• **Massonet, Christoph**
  **52064 Aachen (DE)**
• **Backes, Damian**
  **52070 Aachen (DE)**
• **Seiwert, Paul**
  **52072 Aachen (DE)**
• **Rewitz, Kai**
  **52066 Aachen (DE)**
• **Wesseling, Mark**
  **52062 Aachen (DE)**

(74) Representative: **Markowitz, Markus
Ford-Werke GmbH
Patentabteilung NH/4L
Henry-Ford-Straße 1
50735 Köln (DE)**

(56) References cited:
EP-A1- 3 205 547       DE-A1- 102011 077 993
DE-A1- 102014 224 489  DE-A1- 102015 004 308
DE-A1- 102017 105 871  DE-A1- 102017 200 909
JP-A- 2004 352 108     KR-A- 20160 128 489
US-A- 5 994 701        US-A1- 2006 208 874
US-A1- 2007 033 999    US-A1- 2013 314 536
US-A1- 2018 251 007    US-A1- 2019 061 619

**Description**

The present invention refers to a vehicle, particularly to a vehicle for mass transportation such as a van, bus, train, boat, ferry, ship or air plane. The present invention also refers to a vehicle such as passenger car. Furthermore, the invention refers to a calibration system and a method for function control of a vehicle.

In vehicles for passenger mass transport, such as busses or trains, it is known to provide climate control based on air temperatures, which may be determined in certain areas of the vehicle. This type of climatization control may cause discomfort. The temperature may still be too high or too low for at least some of the passengers. Apart from climatization comfort, vehicles for passenger mass transport may have additional safety and functional limitations, particularly in view of the high number of possible passenger accommodation as well as frequent changes in the number of passengers.

As background state of the art publications DE 10 2017 200909 A1, EP 3 205 547 A1, DE 10 2017 105871 A1, DE 10 2011 077993 A1, US 2018/251007 A1, US 2019/061619 A1, US 2006/208874 A1, DE 10 2014 224489 A1, US 5 994 701 A, US 2013/314536 A1, JP 2004 352108 A, KR 2016 0128489 A and US 2007/033999 A1 are mentioned.

DE 10 2015 004308 discloses a vehicle with a passenger cabin for accommodating a plurality of vehicle passengers, and with a sensor device installed within the passenger cabin, the sensor device being configured for simultaneous and/or sequential detection of at least a plurality of vehicle passengers within the passenger cabin.

Against this background, it has been the objective of the present invention to provide a vehicle for transportation offering more accurate and/or additional vehicle functions, while at the same time limiting costs for vehicle components. It has also been an objective to improve the accuracy of vehicle functions and/or facilitate the precise initiation of vehicle functions. Finally, it has been an objective to provide a method for function control of a vehicle.

As regards the vehicle, at least one of the above-mentioned objectives have been solved by the subject matter of claim 1. A calibration system is subject to claim 11. A method for function control of a vehicle is subject to claim 12. Preferred embodiments are laid down in the dependent claims and will be discussed in the following.

A vehicle for according to the present invention may, in particular, be a van, bus, train, boat, ferry, ship or airplane. A vehicle according to the present invention may therefore be a vehicle for mass transportation. Mass transportation within the meaning of the present invention refers to the transportation of a high number of passengers, particularly more than 5 passengers. The present invention is, however, not limited to these types of vehicles, and may also refer to vehicles other than vehicles for mass transportation. In particular, the present invention may also refer to passenger cars, for example, for the transportation of up to 5 passengers or occupants.

A vehicle according to the present invention comprises a passenger cabin for accommodating a plurality of vehicle passengers, and a sensor device installed within the passenger cabin, the sensor device being configured for simultaneous and/or sequential detection of at least a plurality of vehicle passengers within the passenger cabin.

By providing a sensor device being configured for the detection of a plurality of vehicle passengers, individualized vehicle functions may be provided to and/or for the different passengers. In particular, specific parameters referring to the different passengers may be determined with such sensor device, therewith allowing to provide highly individualized vehicle functions to each of the detected passengers. It is also possible to increase the accuracy of overall vehicle functions dependent on the detection of a plurality of vehicle passengers within the vehicle cabin. At the same time, the number of components may be maintained low, as the detection of the plurality of vehicle passengers by the sensor device may be used for a rather high number of different vehicle functionalities.

In the present context, the detection of at least a plurality of vehicle passengers by the sensor device may include measuring operations with regard to said passengers. Therefore, the term detection not only refers to recognizing the presence of the passengers but also to measurement operations beyond recognizing only the presence of passengers.

In the present context, the term "passenger" may refer to occupants in general, including, for example, a driver, a co-driver, and/or a rear-passenger.

According to a preferred embodiment of the present invention, the sensor device may comprise at least one optical sensor unit for sensing wavelengths in the visible and/or near infrared spectrum and/or at least one temperature sensor unit for sensing wavelengths in the long-wave infrared spectrum. These different sensor units may be combined within one single unit, for example, by arrangement within one casing. It is also possible to arrange these different units separate from each other.

The long-wave infrared spectrum comprises wavelengths between 7 $\mu$m and 15 $\mu$m, particularly between 8 $\mu$m and 14 $\mu$m. Accordingly, long-wave infrared radiation may refer to a radiated heat, preferably - but not limited to - a wavelength of 8 $\mu$m to 14 $\mu$m.

By providing an optical sensor unit for sensing wavelengths in the visible spectrum and a temperature sensor unit for sensing wavelengths in the long-wave infrared spectrum, additional functionality may be provided. The optical sensor unit may, for example, determine the presence of a person and/or localize certain areas of the body of a person. The temperature sensor unit may then determine the temperature of said person within different areas, for example, the skin temperature in the forehead region, or also the surface temperature of the persons clothing. The installation of different sensor units therefore further improves the possibility of providing highly individualized functionalities, such as individualized passenger climate control. According to the present invention, this type of climate control may particularly be provided to a high number of passengers within the passenger cabin.

The sensor device may, according to a further preferred embodiment, be configured for night vision and/or comprise a night vision functionality. The respective detection functionality and/or surveillance functionality may also be provided at darkness and/or faulty lighting. Such functionality may be provided by a near infrared sensor, and may furthermore require an additional near infrared light source.

According to a yet further preferred embodiment, the sensor device may be a condition sensor device for measuring a passenger condition and/or a vehicle cabin condition. The sensor device may be configured to evaluate a plurality of parameters of a set of vehicle interior and/or vehicle passenger parameters, the set of parameters including at least one of:

- a vehicle cabin air temperature parameter,
- a vehicle cabin surface temperature parameter,
- a passenger thermal body state parameter,
- a passenger metabolic rate parameter,
- a passenger clothing level parameter,
- a passenger respiratory rate parameter, and/or
- a passenger mood state parameter.

The above-mentioned passenger and/or vehicle parameters may be evaluated with respect to every detected vehicle passenger and/or within a plurality of areas within the passenger cabin, preferably in proximity of each passenger detected by the sensor device. This allows a further improved degree of individualized vehicle functionality and/or accuracy of vehicle functions.

According to a yet further preferred embodiment, a plurality of sensor devices may be provided. In case of a plurality of sensor devices, each sensor device may be arranged at a different position within the passenger cabin and/or at a distance relative to each other. The accuracy may thereby be further improved. In particular, a higher number of passengers within the passenger cabin may be detected with improved precision. Furthermore, different areas of the passenger cabin, particularly within the proximity of the different passengers, may be measured with increased accuracy.

According to a yet further preferred embodiment, the sensor device may be configured for detection of vehicle passengers on all or at least a majority of passenger accommodation places within the passenger cabin. That is, a comparably high number of passengers within the cabin may be detected with the sensor device, thus, allowing to provide individualized vehicle functionalities to this high number of passengers. The overall comfort and/or safety may therewith be further improved.

According to a yet further preferred embodiment, the passenger cabin may be configured for the accommodation of more than 2 passengers, more than 5 passengers, preferably more than 8 passengers, preferably more than 10 passengers, preferably more than 15 passengers, preferably more than 20 passengers, preferably more than 30 passengers, preferably more than 50 passengers. This allows an efficient passenger transportation, while at the same time ensuring a high degree of comfort and/or safety for the respective passengers.

According to a further preferred embodiment, a passenger accommodation place may be a seat or a standing place. A seat is particularly comfortable for long distant travelling, and a standing place may provide economical transportation by efficiently using the space within the vehicle cabin.

According to a further preferred embodiment, the sensor device may comprise a mechanical and/or optical adjustment. An adjustment possibility further improves the flexibility of detecting a plurality of passengers with one and the same sensor device. Particularly, an adjustment possibility allows to more easily detect a plurality of passengers in a sequential manner, while improving the detection accuracy for each detection process. That is, the sensor device may be adjusted for improved detection accuracy of different detection objectives, such as the detection of different passengers or different vehicle cabin conditions or different vehicle cabin areas.

Preferably, the sensor device may be movably arranged and/or swivel-mounted within the passenger cabin. This allows a reliable adjustment of the sensor device within the cabin with only little constructional effort. The sensor device may, in particular, be configured for panning. Wide areas of the passenger cabin may thereby be covered. Movable arrangement and/or swivel-movements may be realized manually and/or electrically.

According to a further embodiment of the present invention, it may also be possible that the sensor device is fixedly arranged within the passenger cabin, preferably comprising an optical axis being arranged with a permanently fixed angle relative to the passenger cabin. Such arrangement is particularly cost efficient and easy to install, thereby requiring only little effort for assembly and a little number of components.

According to a yet further embodiment, the sensor device may be configured for focusing, preferably for autofocusing. Such focusing functionality may further improve the detection accuracy of passengers in different areas of the vehicle cabin and/or the detection accuracy of different cabin areas.

According to a yet further embodiment, the minimum pixel density of the temperature sensor unit and/or the minimum pixel density of the optical sensor unit is determined on the basis of the distance between the sensor device and the furthermost passenger accommodation place to be detected by the sensor unit and/or on the basis of the distance between the sensor device and the furthermost passenger accommodation place present within the passenger cabin. A high number of passengers or even all passengers within the cabin may thereby be detected with a high detection accuracy. Also, the area of the cabin in proximity of the furthermost passenger accommodation place may be detected with high precision and high reliability.

According to a yet further embodiment, the pixel density of the optical sensor unit is at least 4 times higher, preferably at least 5 times higher, than the pixel density of the temperature sensor unit. Such a difference of pixel densities allows to use a particularly cost-effective temperature sensor unit, while at the same time ensuring sufficiently high resolutions by additionally using optical sensor unit with a significant higher pixel density. Optical sensor units may still be provided at low costs even in case of relatively high pixel densities.

According to a yet further embodiment, the minimum pixel density of the temperature sensor unit may be defined by the function:

$$\frac{pixel}{deg} \mid min = d \; \frac{\pi}{180°} \frac{HP}{WF}$$

where

d = distance between the temperature sensor and a passenger face,
HP = minimum number of horizontal pixels for a face resolution, and
WF = average width of a passenger face.

Alternatively, the minimum pixel density of the temperature sensor unit may be defined by the function:

$$\frac{pixel}{deg} \mid min = d \; \frac{\pi}{180°} \frac{VP}{HF}$$

where

d = distance between the temperature sensor and a passenger face,
VP = minimum number of vertical pixels for a face resolution, and
HF = average height of a passenger face.

In the present context, a preferable range of pixel per deg may be between 0,05 and 10 in horizontal direction.

The above-mentioned distance between the temperature sensor and a passenger face may particularly refer to the distance between the sensor device and a passenger on a furthermost passenger accommodation place to be detected by the sensor device. Thereby, a suitable pixel density of the temperature sensor unit may be determined, allowing an accurate temperature measurement for a high number of passengers within the cabin, while at the same time limiting the cost efforts for the sensor device.

According to a yet further preferred embodiment, the vehicle of the present invention may further comprise a control device for at least one vehicle function. Said control device may preferably be configured to initiate at least one vehicle function based on a sensor signal or a plurality of sensor signals provided by the sensor device. The control device may furthermore be configured to initiate individual and/or automatic functions for at least one or a plurality of passengers within the passenger cabin. Such a control device provides a high degree of flexibility for providing different vehicle functions, particularly coordinated vehicle functions.

According to a yet further preferred embodiment, the sensor device and/or the control device may form an evaluation system for passenger condition evaluation and/or cabin condition evaluation. Such evaluation system may be configured for passenger condition evaluation and/or cabin condition evaluation based on at least one parameter of a set of parameters including:

- at least one environmental parameter of a vehicle interior,
- at least one vehicle passenger physiological parameter, and
- at least one vehicle passenger psychological parameter.

The set of parameters may preferably include:

- a vehicle cabin air temperature parameter,
- a vehicle cabin surface temperature parameter,
- a passenger thermal body state parameter,
- a passenger metabolic rate parameter,
- a passenger clothing level parameter,
- a passenger respiratory rate parameter, and/or
- a passenger mood state parameter.

The above-mentioned parameters may be evaluated by the sensor device and/or the control device. The sensor device may conduct measurements of the cabin interior and/or at least one passenger, and provide signals and/or data to the control device for evaluation, particularly by suitable algorithms.

According to a yet further preferred embodiment, the sensor device may be configured for the detection of seat occupation, seating position and/or the passenger number within the cabin. This allows to provide vehicle functions dependent on the detected seat occupation, seating position and/or number of passengers within the cabin. For example, the sensor device may detect a seat occupation of a specific seat within the vehicle cabin and also the seating position of the respective passenger. Based on this detection, an individualized control of a vehicle function may be provided, particularly to the respective passenger. The sensor device may also be configured for object classification, such as humans, animals and/or things, such as shopping bags and/or luggage.

According to a yet further preferred embodiment, the control device may be configured to initiate individual and/or automatic climatization and/or seat heating and/or cooling for at least one or a plurality of passengers within the cabin. These vehicle functions may preferably be provided dependent on a detected seat occupation and/or seat position of the respective passenger and/or dependent on a detected passenger condition. This allows an efficient control of vehicle functions, and may increase the passenger comfort. The risk of discomfort due to temperatures being too high or too low may be avoided.

According to a yet further preferred embodiment, the control device may be configured to initiate fresh air adjustment, preferably based on detected heat losses, air consumption and/or moisture release within the cabin. The passenger comfort may be further increased in this manner, and climatization be controlled more efficiently.

According to a yet further preferred embodiment, the determination of the necessary heating and/or cooling power within the passenger cabin may be based on a detected number of passengers and/or their metabolism. These factors may have a high influence on the heat flow which has to be balanced with the ambient environment or the fresh air rate for acceptable oxygen and humidity levels.

According to a yet further preferred embodiment, the sensor device and/or the control device may be configured for the temperature measurement of at least one surface within the cabin and/or for thermal preconditioning of at least one surface and/or the cabin air. The measurement of cabin surfaces allows a further improved climatization control, and particularly a suitable preconditioning of surfaces and/or cabin air, thereby allowing further improvement of passenger comfort. Particularly, the preconditioning may ensure passenger comfort already at the time of entering of the vehicle by a passenger.

According to a yet further preferred embodiment, the sensor device and/or the control device may form a safety and/or assist and/or comfort system, preferably for adjusting tire pressure based on the detected number and/or weight and/or position of passengers within the cabin. An optimal tire pressure of a vehicle may depend on the weight of the vehicle and therefore also on the number of passengers within the vehicle. By providing an according configuration of the sensor device and/or the control device, separate sensors for the measurement of a weight loads on the vehicle axles may thereby be omitted, which allows to reduce the number of components and costs. At the same time, an ideal tire pressure may be maintained in view of the vehicle load.

According to a yet further preferred embodiment, the sensor device and/or the control device may form a safety and/or assist and/or comfort system for adapting a damper and/or suspension profile, a steering support profile, an automatic braking calculation, an ESP-setting and/or an ABS-setting based on the detected number and/or weight and/or position of passengers within the cabin.

According to a yet further preferred embodiment, the sensor device and/or the control device may form a safety and/or assist and/or comfort system for the initiation of safety functions based on the detection of passengers in safety areas of the cabin and/or for controlling door functions based on detected passengers when entering or exiting through a door of the cabin. Dangerous clamping of passengers may be avoided and comfort increased by automatic door functions, particularly without providing separate door sensors.

According to a yet further preferred embodiment, the sensor device and/or the control device may form a safety and/or comfort system for the detection of a disabled passenger, for example by detecting a wheelchair, walking frame and/or walking stick, and/or for initiating a service function for disabled passengers. By detecting a disabled passenger, particularly in the door area of the cabin, a service signal may be provided to a service personnel, which may then provide personal assistance to the detected disabled person. The same may apply to service assistance in the seating requirements of a disabled passenger.

According to a yet further preferred embodiment, the sensor device and/or the control device may form a safety and/or assist and/or comfort system for detecting a seating height position and/or initiating an airbag and/or seatbelt tightening based on a detected seating height position. The risk of passenger injuries in case of an accident may thereby be further reduced. That is, depending on the seating height position the airbag initiation and seatbelt tightening may be adjusted. Airbag initiation and seatbelt tightening may thereby also be individualized for the respective passenger within the cabin.

According to a yet further preferred embodiment, the sensor device and/or the control device may be configured for monitoring upcoming and/or actual bad health conditions and/or for alerting in case of any bad health condition, particularly prior to a flight, during boarding, during a flight and/or after landing. According to a yet further preferred embodiment, the sensor device and/or the control device may form a safety camera, preferably with night view functionality and/or for vandalism detection. Surveillance of the passenger cabin may be facilitated and the risk of damages to passengers and/or to the cabin interior reduced.

A further aspect of the present invention likewise refers to a vehicle, in particular a passenger car, bus or train, ship or airplane. A vehicle according to this aspect of the present invention may be configured in accordance to the above description, but is not necessarily limited thereto. In particular, the vehicle to this further aspect may be a vehicle for mass transportation or a vehicle for individual transportation, such as a passenger car for five or less passengers.

A vehicle according to this further aspect of the present invention may comprise a passenger cabin for accommodating a plurality of vehicle passengers, a sensor device installed within the passenger cabin, and at least one calibration target installed within the passenger cabin at a distance from the sensor device. The sensor device may be configured for detection of the calibration target and for calibration based on at least one calibration factor.

A vehicle with such calibration target may allow an accurate calibration of a sensor device with only limited effort and costs, while at the same time ensuring a high operational safety. To the contrary, calibration via internal temperature sensors, particularly temperature sensors within or arranged in direct proximity of the sensor device, may not allow a sufficiently accurate calibration.

Furthermore, the use of a shutter for sensor calibration may be omitted by providing a calibration target in accordance with the present invention. Such shutters may have a known emissivity and may be closed temporarily over the lens of a sensor device. The shutter temperature may be assumed to be equal to the temperature of the sensor device. However, the opening and closing of such shutter may cause noise within the vehicle cabin and wear over time due to frequent opening and closing movements. These drawbacks are solved with the calibration target according to the present invention. The calibration target according to the present invention may be free of any movable parts and enable a precise and reliable calibration in view of suitable calibration factors, particularly related to the measuring area of interest.

According to a yet further preferred embodiment, at least one calibration target may be a calibration surface. Such calibration target may be a visible or a hidden calibration surface. A visible calibration surface may be free of any covering and a hidden calibration target may, for example, be hidden behind a trim cover. A hidden calibration surface may particularly be identified by infrared imaging.

A calibration surface may have known and/or predefined diffuse and/or directional properties. A calibration surface may furthermore have known and/or predefined specular emissivity, transmissivity, and/or reflectivity. Such properties may be taken as a basis for calibration of the sensor device. Filters may, for example, be used for the identification of reflected radiation and/or sun load. A calibration surface may furthermore be integrated into a seat and be configured for passenger detection.

According to a yet further preferred embodiment, at least one calibration target may be an infrared light source. An infrared light source may provide a specifically reliable detection by the sensor device, which may comprise an infrared sensor unit. The operational safety may be further improved.

According to a yet further preferred embodiment, a plurality of calibration targets may be installed within the cabin. Furthermore, the sensor device may be configured for detection of a plurality of calibration targets and for calibration based on calibration factors referring to a plurality of calibration targets. The calibration accuracy may be further increased by a plurality of calibration targets. Single malfunctions of calibration targets would not lead to an entire malfunction of the sensor device, as other calibration targets may ensure a sufficiently accurate calibration. In addition to this, certain calibration measures may be requiring at least two calibration targets for proper calibration. Also, in case a pixel of the sensor device is damaged, particularly a pixel corresponding to a calibration target, a compensation with another calibration target may be achieved.

According to a yet further preferred embodiment, the calibration target may be installed at a distance of more than 10cm, preferably more than 20cm from the sensor device. It is furthermore possible that the calibration target is installed at a distance of more than 50cm, preferably more than 1m or more than 2m from the sensor device. Such distances allow a flexible arrangement of the calibration target within the passenger cabin, particularly in any area of the cabin to be considered important as a measuring area.

According to a yet further preferred embodiment, the calibration factor may be a position and/or orientation of the calibration target within the cabin. A calibration factor may furthermore be a relative distance and/or orientation between the sensor device and the calibration target. Such a calibration factor may be specifically suitable for the calibration of sensor devices being movably arranged and/or swivel-mounted within the passenger cabin. Large areas of the cabin may thereby be covered by the sensor device, while at the same time allowing a precise measurement due to the possibility of accurate calibration.

According to a yet further preferred embodiment, the calibration factor may be a temperature of the calibration target, preferably a surface temperature, and/or radiation temperature of the calibration target. Such calibration factor is particularly suitable for sensor devices comprising a temperature sensor unit, such as an infrared sensor unit. An infrared sensor unit may be configured for long-wave infrared measurement.

According to a yet further preferred embodiment, the sensor device may be configured for temperature calibration and/or for calibration of the sensor orientation and/or position. Temperature calibration may particularly be conducted on the basis of a surface temperature and/or radiation temperature of the calibration target as a calibration factor. The calibration of the position and/or orientation of the sensor device may particularly be conducted on the basis of a position and/or orientation of the calibration target within the cabin and/or a distance between the calibration target and the sensor device as a calibration factor. The temperature calibration and/or calibration of position and/or orientation of the sensor device may be conducted with high accuracy and reliability by applying such calibration factors for the respective calibration operation.

According to a yet further preferred embodiment, the sensor device may be configured for focus calibration based on at least one calibration factor. In other words, the calibration target may provide a focusing reference for the sensor device. The sensor device may be configured for detecting the calibration target, preferably by detection of a known shape of the calibration target by infrared measurement. For this purpose, the calibration target may be forced to a desired cold or warm temperature and/or different surface properties, such as the emissivity, may be taken as a basis for the detection of the calibration target. Subsequently an autofocusing scheme may be conducted. Such autofocusing and/or focus calibration is particularly suitable for far distant applications, for example, for a passenger cabin of a bus, train, ship and/or airplane.

According to a yet further preferred embodiment, the sensor device may be configured for detecting at least one calibration factor by measurement and/or by signal transmission from at least one calibration target to the sensor device. For example, the temperature present within a calibration target may be transmitted, wire based and/or wireless, to the sensor device, and based on this transmitted temperature value a suitable temperature calibration of the sensor device may be conducted.

According to a yet further preferred embodiment, at least one calibration factor may be predefined and/or preset within the sensor device and/or the calibration target. For example, the spectral properties of a calibration surface in the spectral range of interest may be known and preset within the sensor device. Dependent on these predefined spectral properties, a suitable temperature calibration of the sensor device may be conducted. It is, for example, also possible that the shape of a calibration surface is known and predefined within the sensor device. The sensor device may therefore comprise specific data on at least one or more calibration targets.

According to a yet further preferred embodiment, the sensor device may be configured for detecting the calibration target by shape and/or temperature difference, preferably temperature difference relative to other and/or adjacent cabin surfaces. This allows a reliable detection of the calibration target by the sensor device, and therewith also a reliable calibration of the sensor device.

According to a yet further preferred embodiment, the sensor device may be configured for detecting the calibration target by at least one spectral property of a calibration surface, preferably a spectral property within a predefined wavelength band. The respective spectral property may preferably be a reflectivity, absorptivity and/or emissivity.

According to a yet further preferred embodiment, the sensor device may be configured to determine a sun load and/or incident radiation based on at least one surface property of a calibration surface, preferably a spectral property within a predefined wavelength band and/or a reflectivity, absorptivity and/or emissivity. The influence of sun load and/or incident radiation may therefore be used as a parameter for the initiation of a vehicle function, such as a climatization function.

According to a yet further preferred embodiment, the calibration target may comprise at least one temperature measuring device, preferably integrated and/or embedded within the surface of a calibration target. The temperature measuring device may, for example, be a thermistor, preferably an NTC thermistor. Furthermore, the calibration target may be configured to provide measurement signals of an integrated temperature measuring device to the sensor device for temperature calibration. This allows for the precise control of the calibration target, and thus allows an accurate calibration of the sensor device.

According to a yet further preferred embodiment, the calibration target may comprise a heating and/or cooling device for adjusting and/or controlling the temperature of the calibration target, preferably a surface of the calibration target facing the cabin. A desired temperature of the calibration target may thus be maintained at high precision. A heating and/or cooling device of the calibration may preferably comprise a thermoelectric unit and/or a ventilation unit. Such thermoelectric unit and/or a ventilation unit may provide a reliable temperature regulation, and may at the same time be provided with only limited cost effort.

According to a yet further preferred embodiment, at least one calibration target may be a temperature-controlled system, preferably a heated and/or cooled seat, a backlight and/or heated and/or cooled cabin surfaces. Such temperature-controlled systems may be available in vehicles independently of a sensor device according to the present invention, but in addition used for the calibration of a sensor device. Thus, the number of components may be maintained low, and the sensor calibration be enabled in a cost-efficient manner.

A further independent aspect of the present invention refers to a calibration system according to claim 11. The sensor device and the calibration target may be two separate and/or independently installable components or units. According to this aspect, the sensor device may furthermore be configured for detection of the calibration target and for calibration based on at least one calibration factor.

A yet further independent aspect of the present invention refers to a method for function control of a vehicle, preferably of a vehicle according to the above description, according to claim 12.

A yet further independent aspect of the present disclosure refers to a method for calibration of a sensor device of a vehicle, preferably of a vehicle according to the above description. The method may comprise the steps of detecting at least one calibration target by a sensor device, the calibration target and the sensor device being installed within the passenger cabin at a distance from each other, and calibrating the sensor device based on at least one calibration factor, preferably referring to the detected calibration target.

The above explanations and details regarding the vehicle for mass transportation and/or regarding the vehicle comprising the calibration target may also apply to the calibration system, the method for function control of a vehicle and/or the method for calibration of a sensor device of a vehicle.

Other embodiments of the invention result from combinations of the features disclosed in the claims, the specification and the drawings.

Fig. 1      is a schematic diagram of a vehicle according to an embodiment of the present invention,

Fig. 2      is a schematic diagram of a vehicle according to a further embodiment of the present invention,

Fig. 3      is a schematic diagram of a vehicle according to a further embodiment of the present invention,

Fig. 4      is a schematic diagram of a vehicle according to a further embodiment of the present invention,

Fig. 5a      shows an exemplary visual view of a sensor device according to an embodiment of the present invention,

Fig. 5b      shows an exemplary infrared view of a sensor device according to an embodiment of the present invention,

Fig. 6      shows a first focus configuration of a sensor device in the vehicle of fig. 4,

Fig. 7      shows a second focus configuration of a sensor device in the vehicle of fig. 4,

Fig. 8      shows a third focus configuration of a sensor device in the vehicle of fig. 4.

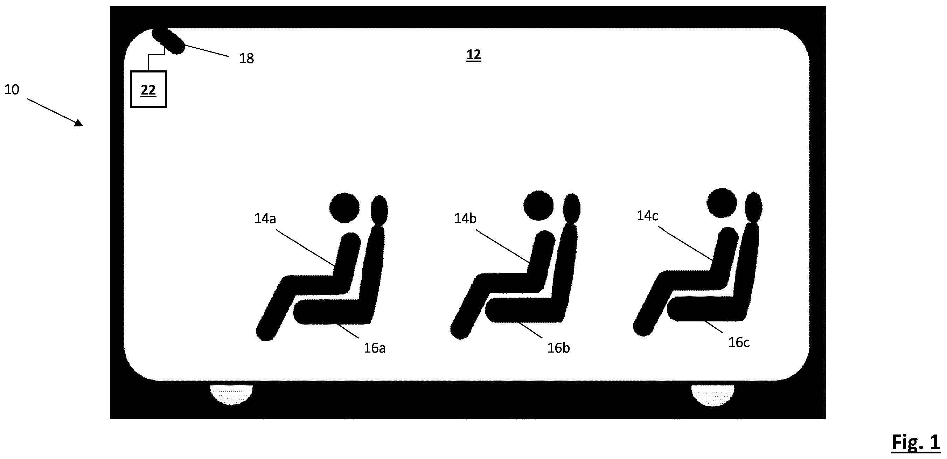

Fig. 1 is a schematic diagram of a vehicle 10 according to an embodiment of the present invention. The vehicle 10 may particularly be a vehicle for mass transportation, such as a bus or train. The vehicle 10 may comprise a passenger cabin 12 for accommodating a plurality of vehicle passengers 14. The passenger cabin 12 may comprise a plurality of accommodation places, such as seats 16 for the passengers 14a to 14c. In the present embodiment, the cabin 12 may be equipped with three rows of seats 16a to 16c for the accommodation of passengers 14a to 14c.

The vehicle 10 shown in fig. 1 may further comprise a sensor device 18 device installed within the passenger cabin 12. The sensor device 18 may, for example, be installed at or close to a roof of the passenger cabin 12. Furthermore, the sensor device 18 may comprise at least one optical sensor unit for sensing wavelength in the visible spectrum and/or at least one temperature sensor unit for sensing wavelengths in the long-wave infrared spectrum. The different sensor units of the sensor device 18 are not shown here in detail. The sensor device 18 may be a condition sensor device for measuring a passenger condition and/or a vehicle cabin condition.

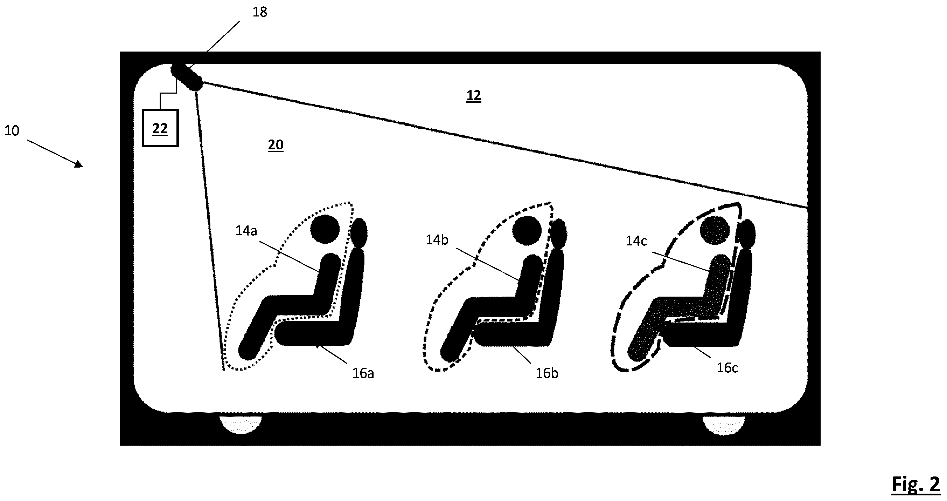

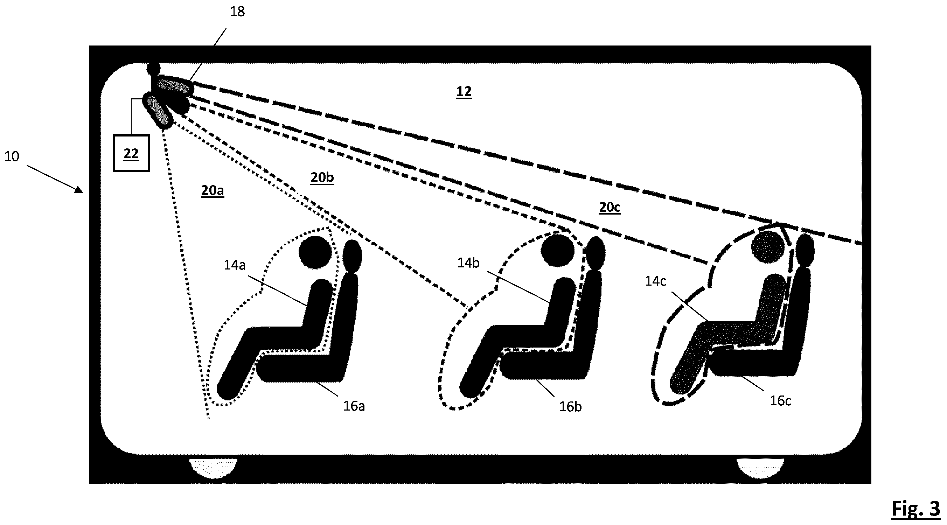

As will become more apparent with reference to fig. 2 and 3, the sensor device 18 may be configured for simultaneous and/or sequential detection of at least a plurality of vehicle passengers 14a to 14c within the passenger cabin 12. The sensor device 18 may particularly be configured for detection of vehicle passengers 14a to 14c on all or at least a majority of passenger accommodation places within the passenger cabin 12, such as on the seats 16a to 16c.

According to the embodiment in fig. 2, the sensor device 18 may be fixedly arranged within the passenger cabin 12. The sensor device 18 may particularly have an optical axis being arranged with a permanently fixed angle relative to the passenger cabin 12. At the same time, the sensor device 18 shown in fig. 2 may have a comparatively large field of view 20. The field of view 20 may, for example, cover all passengers 14a to 14c within the cabin 12. Accordingly, the sensor device 18 of fig. 2 may be configured for multi area detection, particularly for detection in areas comprising a plurality of seats 16a to 16c.

Furthermore, according to the embodiment in fig. 2, the sensor device 18 may be configured for simultaneous and/or sequential detection of a plurality of vehicle passengers 14a to 14c. That is, the sensor device 18 may detect a plurality of passengers 14 with one single focus configuration at the same time, or detect different passengers sequentially with different focus configurations applied subsequent to each other. Accordingly, the sensor device 18 in the embodiment of fig. 2 may be configured for focusing, preferably for autofocusing. The focusing enables a suitable optical adjustment.

According to the embodiment in fig. 3, the sensor device 18 may comprise a mechanical adjustment. That is, the sensor device 18 may be movably arranged and/or swivel-mounted within the passenger cabin 12. The sensor device 18 in fig. 3 may particularly be configured for panning. The plurality of passengers 14a to 14c may be detected sequentially by moving the sensor device 18 into different swivel positions. At the same time, also the sensor device 18 in the embodiment of fig. 3 may be configured for focusing, preferably for autofocusing.

According to a first position shown in fig. 3, the passenger 14a may be detected by the sensor device 18. In this first position, the sensor device 18 may have a field of view 20a. The field of view 20a may not only be defined by the swivel position of the sensor device 18, but also by its focus configuration.

According to a second position shown in fig. 3, the passenger 14b may be detected by the sensor device 18. In this second position, the sensor device 18 may have a field of view 20b. The field of view 20b may not only be defined by the swivel position of the sensor device 18, but also by its focus configuration.

According to a third position shown in fig. 3, the passenger 14c may be detected by the sensor device 18. In this third position, the sensor device 18 may have a field of view 20c. The field of view 20c may not only be defined by the swivel position of the sensor device 18, but also by its focus configuration.

The minimum pixel density of the temperature sensor unit and/or the minimum pixel density of the optical sensor unit of the sensor device 18 may be determined on the basis of the distance between the sensor device 18 and the furthermost seat 16c accommodating the furthermost passenger 14c to be detected by the sensor unit. Furthermore, the pixel density of the optical sensor unit may be at least 4 times higher, preferably at least 5 times higher, than the pixel density of the temperature sensor unit of the sensor device 18. The minimum pixel density of the temperature sensor unit may be defined by the function:

$$\frac{pixel}{deg} \;\Big|\min = d\;\frac{\pi}{180°}\;\frac{\text{HP}}{\text{WF}}$$

where

d = distance between the temperature sensor and a passenger face,
HP = minimum number of horizontal pixels for a face resolution, and
WF = average width of a passenger face.

As may be comprehended from fig. 1 to 3, the vehicle 10 may further comprise a control device 22 for at least one vehicle function. The control device 22 may preferably be configured to initiate at least one vehicle function on the basis of a sensor signal or a plurality of sensor signals provided by the sensor device 18. The control device 22 may particularly be configured to initiate individual and/or automatic functions for at least one or a plurality of passengers 14a to 14c within the passenger cabin.

The sensor device 18 and the control device 22 may form an evaluation system for passenger condition evaluation and/or cabin condition evaluation. The sensor device 18 may be configured for the detection of an occupation of at least one seat 16a to 16c, a seating position and/or a number of passengers 14a to 14c within the cabin 12. The control device 22 may furthermore be configured to initiate individual and/or automatic climatization and/or seat heating and/or cooling for at least one or a plurality of passengers 14a to 14c within the cabin 12. It is also possible that the control device 22 is configured for fresh air adjustment, preferably based on detected heat losses, air consumption and/or moisture release. The sensor device 18 and/or the control device 22 may be configured for the temperature measurement of at least one surface within the cabin 12 and/or for thermal preconditioning of at least one surface and/or the cabin air.

Furthermore, the sensor device 18 and/or the control device 22 may form a safety and/or comfort system, preferably for adjusting tire pressure based on the detected number and/or weight and/or position of passengers 14a to 14c within the cabin 12 and/or for the initiation of safety functions based on the detection of passengers 14a to 14c in safety areas of the cabin 12. It is also possible that the sensor device 18 and/or the control device 22 is configured for controlling door functions based on detected passengers 14a to 14c when entering or exiting through a door of the cabin 12 and/or for the detection of disabled passengers 14a to 14c and/or for initiating a service function for disabled passengers 14a to 14c. The sensor device 18 and/or the control device 22 may also be configured for detecting a seating height position and/or initiating an airbag and/or seatbelt tightening based on a detected seating height position.

The sensor device 18 and/or the control device 22 of figs. 1 to 3 may form a safety camera, preferably with night view functionality and/or for vandalism detection.

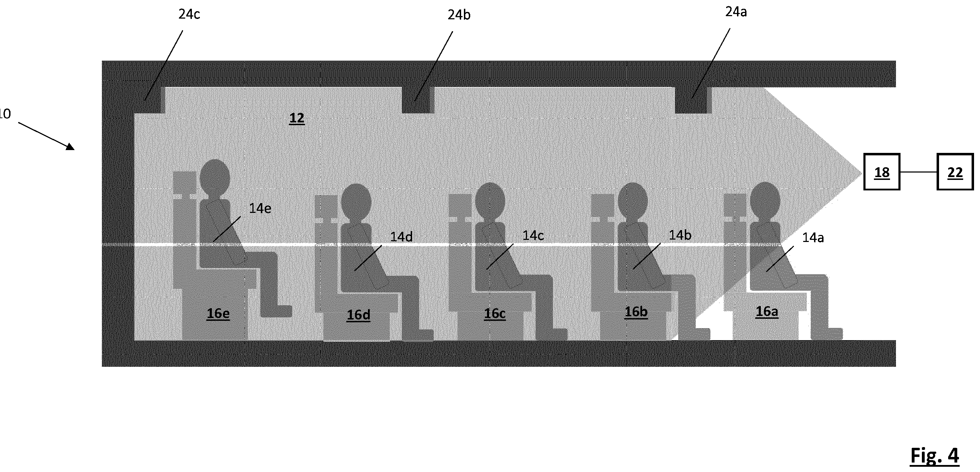

Fig. 4 to 8 refer to a vehicle 10 according to a further embodiment of the present invention. As may be comprehended from fig. 4, the vehicle 10, which may particularly be a passenger car, bus or train, comprises a passenger cabin 12 for accommodating a plurality of vehicle passengers 14a to 14e. The vehicle 10 of fig. 4 furthermore comprises a sensor device 18 installed within the passenger cabin 12, and at least one calibration target 24 installed within the passenger cabin 12 at a distance from the sensor device 18. The sensor device 18 may be configured for detection of the calibration target 24 and for calibration based on at least one calibration factor.

As shown in fig. 4, the vehicle 10 may particularly comprise a plurality of calibration targets 24a to 24c, each being located at a different distance to the sensor device 18 within the passenger cabin 12. Accordingly, the sensor device 18 may be configured for detection of a plurality of calibration targets 24a to 24c. Furthermore, the sensor device 18 may be configured for calibration based on calibration factors referring to one calibration target 24 or referring to a plurality of calibration targets 24a to 24c. Calibration factors of different calibration targets 24a to 24c may be detected by and/or provided to the sensor device 18 for calibration purposes subsequently and/or simultaneously. It is furthermore possible that different calibration factors from one calibration target 24 are detected by and/or provided to the sensor device 18 for calibration purposes.

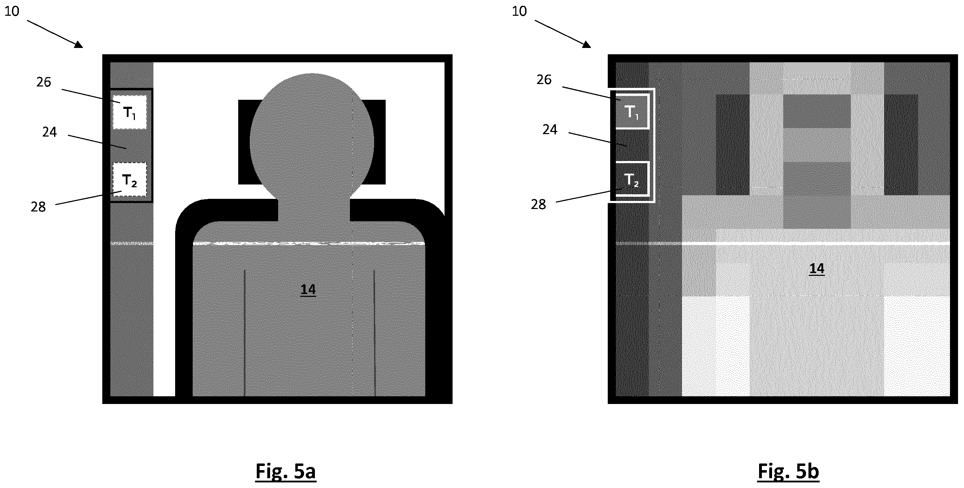

At least one of the calibration targets 24a to 24c may be and/or comprise a calibration surface or a plurality of calibration surfaces, as will be described in greater detail with reference to figs. 5a and 5b. It is also possible that at least one calibration target 24a to 24c is and/or comprises an infrared light source.

A calibration factor may, for example, be a position and/or orientation of the calibration target 24 within the cabin 12 and/or the relative distance and/or orientation between the sensor device 18 and the calibration target 24. Furthermore, a calibration factor may be a temperature of the calibration target 24, preferably a surface temperature, and/or a radiation temperature of the calibration target 24. One calibration target 24 may also provide different calibration factors in terms of different temperatures.

The sensor device 18 may be configured for temperature calibration and/or for calibration of the sensor orientation and/or position. The sensor device 18 may furthermore be configured for focus calibration, preferably in a far distant application, based on at least one calibration factor, as will also be described in greater detail with reference to figs. 5a and 5b.

The sensor device 18 may be configured for detecting at least one calibration factor by measurement and/or by signal transmission from at least one calibration target 24a to 24c to the sensor device 18. It is also possible that at least one calibration factor is predefined and/or preset within the sensor device 18 and/or at within at least one of the calibration targets 24a to 24c.

Furthermore, the sensor device 18 may be configured for detecting the calibration targets 24a to 24c by shape and/or temperature difference, preferably temperature difference relative to other and/or adjacent cabin surfaces. The sensor device 18 may also be configured for detecting a calibration target 24 by temperature difference between two different calibration surfaces of the same calibration target 24.

The sensor device 18 may also be configured for detecting the calibration targets 24a to 24c by at least one spectral property of a calibration surface, preferably a spectral property within a predefined wavelength band. Such spectral property may be preferably a reflectivity, absorptivity and/or emissivity.

At least one of the calibration targets 24a to 24c may be installed at a distance of more than 10cm, preferably more than 20cm, from the sensor device 18. More preferably, each calibration target 24a to 24c may be installed at a distance of more than 10 cm or more than 20cm from the sensor device 18. Further preferred, the at least one of the calibration targets 24a to 24c may be installed at a distance of more than 1m, preferably more than 2m or more than 5m, from the sensor device 18.

Fig. 5a shows an exemplary visual view from the sensor device 18 into the cabin 12 of vehicle 10, and fig. 5b shows an exemplary infrared view of the sensor device 18 into the cabin 12 of vehicle 10. It may be comprehended from fig. 5a and 5b that the view from the sensor device 18 into the cabin 12 of a vehicle 10 according to fig. 4 is inter alia directed to a passenger 14 as well as a calibration target 24.

The calibration target 24 shown in fig. 5a and 5b may comprise two different calibration surfaces 26 and 28. The calibration surfaces 26 and 28 may either be visible or hidden under a trim cover and then only seen in IR imaging. The calibration surface 26 may be at a temperature T1 and the calibration surface 28 may be at a temperature T2. In the present example, the calibration surface 26 may have a higher temperature than the calibration surface 28, thus T1 being higher than T2.

The calibration surface 26 may be temperature controlled. The calibration surface 26 may therefore be heated and/or cooled. Accordingly, the calibration target 24 may comprise a heating and/or cooling device for adjusting and/or controlling the temperature of the calibration surface 26. Such heating and/or cooling device may preferably comprise a thermoelectric unit and/or a ventilation unit. The calibration surface 28 may be free of any temperature control, and therefore temperature T2 may be mainly the result of the environment temperature such as the cabin air and/or radiation. It is also possible that the calibration surface 28 is temperature controlled.

Both calibration surfaces 26 and 28 may be temperature measured. For this purpose, the calibration target 24 may comprise at least one temperature measuring device or a plurality of temperature measuring devices. Such a measuring device may preferably be integrated and/or embedded within a calibration surface 26 or 28 of the calibration target 24. A temperature measuring device may be a thermistor, preferably an NTC thermistor, and/or a thermopile. It is particularly possible that different types of temperature measurement devices or different means of temperature measurement are applied for the measurement of temperatures T1 and T2 of the calibration surfaces 26 and 28. The calibration target 24 may be configured to provide measurement signals of an integrated temperature measuring device to the sensor device 18 for temperature calibration.

It is furthermore possible that the calibration target 24 shown in fig. 5a and 5b, or at least one of the calibration targets 24a to 24c is a temperature-controlled system such as a heated and/or cooled seat, a backlight and/or heated and/or cooled cabin surfaces. The calibration surfaces 26 and 28 may also be integrated into seats 16a to 16e and used for occupant detection.

As may further be comprehended from fig. 5a and 5b, the location of the calibration surfaces 26 and 28, and the expected temperatures T1 and T2 allow the system to calibrate to known temperatures T1 and T2, as well as move or adjust the sensor device 18 to match the expected pixel to the correct position. In case an expected outline is known, such as an outline of a temperature T1 and T2, a lens of the sensor device 18 can be adjusted to the correct focus plane. A calibration surface 26 and 28 may be smaller, same size, or larger than a pixel at the respective focus plane. As may be seen from fig. 5b, the calibration surfaces 26 and 28 may particularly have the same size as a pixel at the respective focus plane.

Based on the detected temperatures T1 and T2 a suitable calibration of the sensor device 18 may be conducted, and, for example, an accurate temperature measurement of the skin of a passenger 14 be conducted.

The calibration surfaces 26 and 28 may have known diffuse and/or directional properties. The calibration surfaces 26 and 28 may also have a known specular emissivity, transmissivity, and/or reflectivity. Filters and other means may be applied in order to determine reflected radiation for sun load and/or incident radiation. Accordingly, the sensor device 18 may be configured to determine a sun load and/or incident radiation based on at least one surface property of a calibration surface 26 or 28, preferably a spectral property within a predefined wavelength band and/or a reflectivity, absorptivity and/or emissivity.

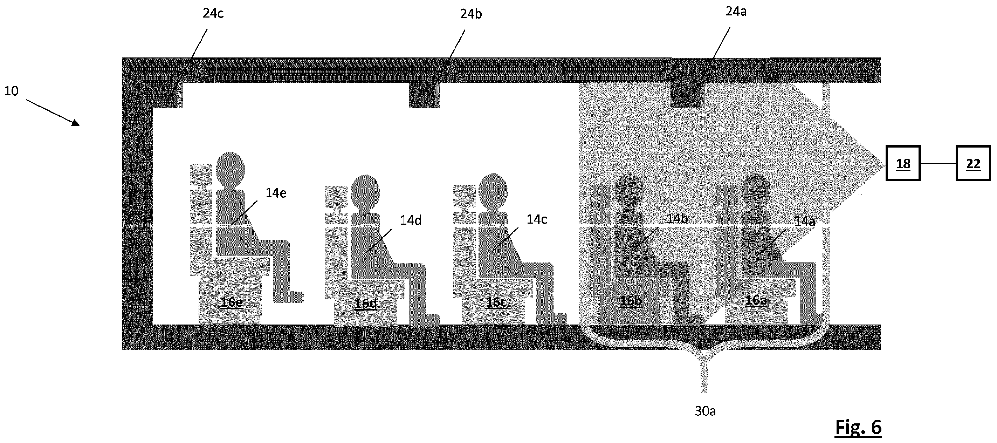

Fig. 6 to 8 shows three different focus configurations of a sensor device 18 in the vehicle 10 of fig. 4. As may be comprehended from fig. 6, the sensor device 18 has a first focus configuration. In this first focus configuration, the sensor device 18 is focused on the calibration target 24a. The calibration target 24a is in proximity to passengers 14a and 14b, and the seats 16a and 16b, respectively. The sensor device 18 has an acceptable focus range 30a, which covers passengers 14a and 14b, and the seats 16a and 16b, respectively. The acceptable focus range 30a may be an acceptable depth of field. In this first focus configuration, a suitable calibration of the sensor device 18 may be conducted, and based on this calibration measurements with regard to passengers 14a and 14b, and seats 16a and 16b may be conducted with high accuracy. Subsequent to calibration in the first focus configuration, and measurements conducted by the sensor device 18 with this calibration, suitable vehicle functions, such as climatization, seat heating and/or cooling or the like, may be initiated with regard to passengers 14a and 14b, seats 16a and 16b, and/or in the area of seats 16a and 16b.

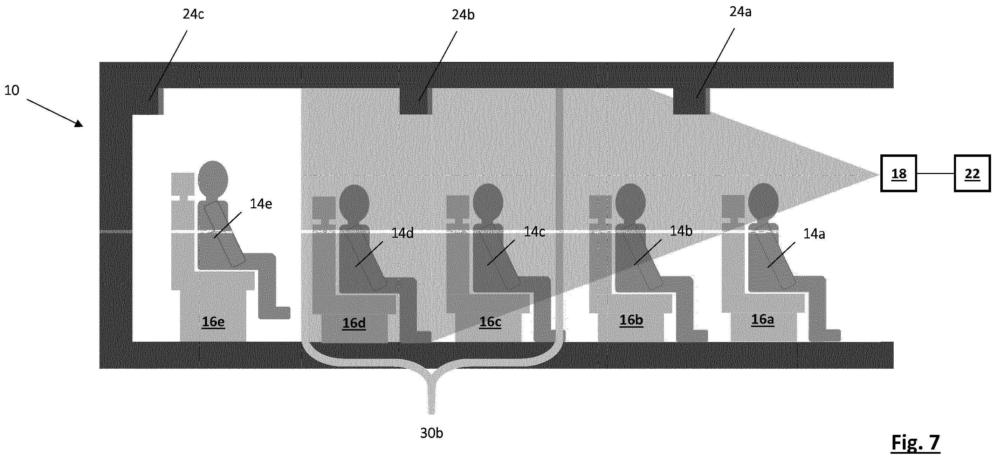

As may be comprehended from fig. 7, the sensor device 18 has a second focus configuration. In this second focus configuration, the sensor device 18 is focused on the calibration target 24b. The calibration target 24b is in proximity to passengers 14c and 14d, and the seats 16c and 16d, respectively. The sensor device 18 has an acceptable focus range 30b, which covers passengers 14c and 14d, and the seats 16c and 16d, respectively. The acceptable focus range 30b may be an acceptable depth of field. In this second focus configuration, a suitable calibration of the sensor device 18 may be conducted, and based on this calibration measurements with regard to passengers 14c and 14d, and seats 16c and 16d may be conducted with high accuracy. Subsequent to calibration in the second focus configuration, and measurements conducted by the sensor device 18 with this calibration, suitable vehicle functions, such as climatization, seat heating and/or cooling or the like, may be initiated with regard to passengers 14c and 14d, seats 16c and 16d, and/or in the area of seats 16c and 16d.

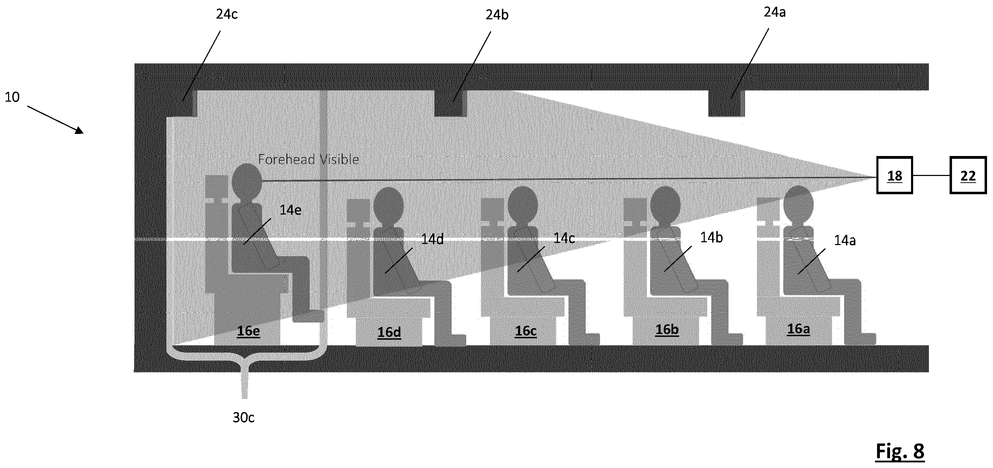

As may be comprehended from fig. 8, the sensor device 18 has a third focus configuration. In this third focus configuration, the sensor device 18 is focused on the calibration target 24c. The calibration target 24c is in proximity to passenger 14e, and seat 16e, respectively. The sensor device 18 has an acceptable focus range 30c, which covers passenger 14e and seats 16e, respectively. The acceptable focus range 30c may be an acceptable depth of field. In this third focus configuration, a suitable calibration of the sensor device 18 may be conducted, and based on this calibration measurements with regard to passenger 14e and seat 16e may be conducted with high accuracy. Subsequent to calibration in the third focus configuration, and measurements conducted by the sensor device 18 with this calibration, suitable vehicle functions, such as climatization, seat heating and/or cooling or the like, may be initiated with regard to passenger 14e, seat 16e and/or in the area of seat 16e.

## Claims

1. Vehicle (10), with a passenger cabin (12) for accommodating a plurality of vehicle passengers (14), and with a sensor device (18) installed within the passenger cabin (12), the sensor device (18) being configured for simultaneous and/or sequential detection of at least a plurality of vehicle passengers (14) within the passenger cabin (12), wherein the sensor device (18) is movably arranged,

   the vehicle further comprising a control device (22) for at least one vehicle function, wherein said control device (22) is configured to initiate at least one vehicle function on the basis of a sensor signal or a plurality of sensor signals provided by the sensor device (18) and wherein the control device (22) is configured to initiate individual and/or automatic functions for at least one or a plurality of passengers (14) within the passenger cabin (12)
   and wherein at least one calibration target (24) is installed within the passenger cabin (12) at a distance from the sensor device (18), wherein the sensor device (18) is configured for detection of the calibration target (24) and for calibration based on at least one calibration factor.

2. Vehicle (10) according to claim 1, wherein the sensor device (18) comprises at least one optical sensor unit for sensing wavelength in the visible and/or near infrared spectrum and/or at least one temperature sensor unit for sensing wavelengths in the long-wave infrared spectrum.

3. Vehicle (10) according to claim 1 or 2, wherein the sensor device (18) is configured for detection of vehicle passengers (14) on all or at least a majority of passenger accommodation places within the passenger cabin (12).

4. Vehicle (10) according to one of the preceding claims, wherein the at least one calibration target (24) comprises a calibration surface (26, 28), wherein a plurality of calibration targets (24) are installed within the cabin (12), and wherein the sensor device (18) is configured for detection of a plurality of calibration targets (24) and for calibration based on calibration factors referring to a plurality of calibration targets (24).

5. Vehicle (10) according to claim 4, wherein a calibration factor is a position and/or orientation of the calibration target (24) within the cabin (12), and/or wherein a calibration factor is a temperature of the calibration target (24).

6. Vehicle (10) according to one of claims 4 or 5, wherein the sensor device (18) is configured for detecting the calibration target (24) by shape and/or temperature difference.

7. Vehicle (10) according to one of claims 4 to 6, wherein the sensor device (18) is configured to determine a sun load and/or incident radiation based on at least one surface property of a calibration surface (26, 28).

8. Vehicle (10) according to one of claims 4 to 7, wherein the calibration target (24) comprises at least one temperature measuring device.

9. Vehicle (10) according to one of claims 4 to 8, wherein the calibration target (24) comprises a heating and/or cooling device for adjusting and/or controlling the temperature of the calibration target (24).

10. Vehicle (10) according to one of claims 4 to 9, wherein at least one calibration target (24) is a temperature-controlled system.

11. Calibration system installed in a vehicle (10),

    the calibration system comprising a sensor device (18) and at least one calibration target (24), the vehicle (10) comprising a passenger cabin (12) for accommodating a plurality of vehicle passengers (14), and a control device (22) for at least one vehicle function, wherein said control device (22) is configured to initiate at least one vehicle function on the basis of a sensor signal or a plurality of sensor signals provided by the sensor device (18) of the calibration system and wherein the control device (22) is configured to initiate individual and/or automatic functions for at least one or a plurality of passengers (14) within the passenger cabin (12),
    wherein the sensor device (18) is installed movably arranged within the passenger cabin (12) of the vehicle (10), the sensor device (18) being configured for simultaneous and/or sequential detection of at least a plurality of vehicle passengers (14) within the passenger cabin (12) and wherein the at least one calibration target (24) is installed within the passenger cabin (12) at a distance from the sensor device (18), wherein the sensor device (18) is configured for detection of the calibration target (24) and for calibration based on at least one calibration factor.

12. Method for function control of a vehicle (10) according to one of the claims 1 to 10, the method comprising the steps of:

    - simultaneous and/or sequential detection of a plurality of vehicle passengers (14) within a passenger cabin (12),
    - initiation of individualized vehicle functions for each of the passengers (14) within the passenger cabin (12) based on detection results,
    - detection of at least one calibration target (24) by the sensor device (18), the calibration target (24) and the sensor device (18) being installed within the passenger cabin (12) at a distance from each other, wherein the sensor device (18) is movably arranged,
    - calibration of the sensor device (18) based on at least one calibration factor referring to the detected calibration target (24).

**Patentansprüche**

1. Fahrzeug (10), mit einem Fahrgastraum (12) zur Aufnahme einer Mehrzahl von Fahrgästen (14) sowie mit einer innerhalb des Fahrgastraums (12) installierten Sensorvorrichtung (18), wobei die Sensorvorrichtung (18) zur gleichzeitigen und/oder sequentiellen Erfassung zumindest einer Mehrzahl von Fahrgästen (14) innerhalb des Fahrgastraums (12) ausgelegt ist, wobei die Sensorvorrichtung (18) beweglich angeordnet ist,

    wobei das Fahrzeug ferner eine Steuervorrichtung (22) für mindestens eine Fahrzeugfunktion umfasst, wobei die Steuervorrichtung (22) ausgelegt ist, mindestens eine Fahrzeugfunktion auf Grundlage eines Sensorsignals oder einer Mehrzahl von Sensorsignalen auszulösen, die durch die Sensorvorrichtung (18) bereitgestellt werden, und wobei die Steuervorrichtung (22) ausgelegt ist, einzelne und/oder automatische Funktionen für mindestens einen oder eine Mehrzahl von Fahrgästen (14) innerhalb des Fahrgastraums (12) auszulösen, und wobei mindestens ein Kalibrierziel (24) innerhalb des Fahrgastraums (12) in einem Abstand zur Sensorvorrichtung (18) installiert ist, wobei die Sensorvorrichtung (18) zur Erfassung des Kalibrierziels (24) und zur Kalibrierung auf Grundlage mindestens eines Kalibrierfaktors ausgelegt ist.

2. Fahrzeug (10) nach Anspruch 1, wobei die Sensorvorrichtung (18) mindestens eine optische Sensoreinheit zur Erfassung von Wellenlängen im sichtbaren und/oder nahinfraroten Spektrum und/oder mindestens eine Temperatursensoreinheit zur Erfassung von Wellenlängen im langwelligen Infrarotspektrum umfasst.

3. Fahrzeug (10) nach Anspruch 1 oder 2, wobei die Sensorvorrichtung (18) zur Erfassung von Fahrgästen (14) auf allen oder zumindest der Mehrheit von Fahrgastplätzen innerhalb des Fahrgastraums (12) ausgelegt ist.

4. Fahrzeug (10) nach einem der vorhergehenden Ansprüche, wobei das mindestens eine Kalibrierziel (24) eine Kalibrieroberfläche (26, 28) umfasst, wobei eine Mehrzahl von Kalibrierzielen (24) innerhalb des Fahrgastraums (12) installiert ist und wobei die Sensorvorrichtung (18) zur Erfassung einer Mehrzahl von Kalibrierzielen (24) und zur Kalibrierung auf Grundlage von Kalibrierfaktoren bezüglich einer Mehrzahl von Kalibrierzielen (24) ausgelegt ist.

5. Fahrzeug (10) nach Anspruch 4, wobei ein Kalibrierfaktor eine Position und/oder Orientierung des Kalibrierziels (24) innerhalb des Fahrgastraums (12) ist und/oder wobei ein Kalibrierfaktor eine Temperatur des Kalibrierziels (24) ist.

6. Fahrzeug (10) nach einem der Ansprüche 4 oder 5, wobei die Sensorvorrichtung (18) zur Erfassung des Kalibrierziels (24) anhand von Form und/oder Temperaturdifferenz ausgelegt ist.

7. Fahrzeug (10) nach einem der Ansprüche 4 bis 6, wobei die Sensorvorrichtung (18) ausgelegt ist, eine Sonneneinstrahlung und/oder einfallende Strahlung auf Grundlage mindestens einer Oberflächeneigenschaft einer Kalibrieroberfläche (26, 28) zu bestimmen.

8. Fahrzeug (10) nach einem der Ansprüche 4 bis 7, wobei das Kalibrierziel (24) mindestens eine Temperaturmessvorrichtung umfasst.

9. Fahrzeug (10) nach einem der Ansprüche 4 bis 8, wobei das Kalibrierziel (24) eine Heiz- und/oder Kühlvorrichtung zur Einstellung und/oder Steuerung der Temperatur des Kalibrierziels (24) umfasst.

10. Fahrzeug (10) nach einem der Ansprüche 4 bis 9, wobei mindestens ein Kalibrierziel (24) ein temperaturgesteuertes System ist.

11. Kalibriersystem, installiert in einem Fahrzeug (10),

wobei das Kalibriersystem eine Sensorvorrichtung (18) und mindestens ein Kalibrierziel (24) umfasst, wobei das Fahrzeug (10) einen Fahrgastraum (12) zur Aufnahme einer Mehrzahl von Fahrgästen (14) sowie eine Steuervorrichtung (22) für mindestens eine Fahrzeugfunktion umfasst, wobei die Steuervorrichtung (22) ausgelegt ist, mindestens eine Fahrzeugfunktion auf Grundlage eines Sensorsignals oder einer Mehrzahl von Sensorsignalen auszulösen, die durch die Sensorvorrichtung (18) des Kalibriersystems bereitgestellt werden, und wobei die Steuervorrichtung (22) ausgelegt ist, einzelne und/oder automatische Funktionen für mindestens einen oder eine Mehrzahl von Fahrgästen (14) innerhalb des Fahrgastraums (12) auszulösen, wobei die Sensorvorrichtung (18) beweglich innerhalb des Fahrgastraums (12) des Fahrzeugs (10) installiert ist, wobei die Sensorvorrichtung (18) zur gleichzeitigen und/oder sequentiellen Erfassung zumindest einer Mehrzahl von Fahrgästen (14) innerhalb des Fahrgastraums (12) ausgelegt ist, und wobei das mindestens eine Kalibrierziel (24) innerhalb des Fahrgastraums (12) in einem Abstand zur Sensorvorrichtung (18) installiert ist, wobei die Sensorvorrichtung (18) zur Erfassung des Kalibrierziels (24) und zur Kalibrierung auf Grundlage mindestens eines Kalibrierfaktors ausgelegt ist.

12. Verfahren zur Funktionssteuerung eines Fahrzeugs (10) nach einem der Ansprüche 1 bis 10, wobei das Verfahren die Schritte umfasst:

- gleichzeitiges und/oder sequentielles Erfassen einer Mehrzahl von Fahrgästen (14) innerhalb eines Fahrgastraums (12),
- Auslösen individualisierter Fahrzeugfunktionen für jeden der Fahrgäste (14) innerhalb des Fahrgastraums (12) auf Grundlage von Erfassungsergebnissen,

- Erfassen mindestens eines Kalibrierziels (24) durch die Sensorvorrichtung (18), wobei das Kalibrierziel (24) und die Sensorvorrichtung (18) innerhalb des Fahrgastraums (12) in einem Abstand voneinander installiert sind, wobei die Sensorvorrichtung (18) beweglich angeordnet ist,

- Kalibrieren der Sensorvorrichtung (18) auf Grundlage mindestens eines Kalibrierfaktors bezüglich des erfassten Kalibrierziels (24).

**Revendications**

1. Véhicule (10), pourvu d'un habitacle passagers (12) destiné à accueillir une pluralité de passagers (14) de véhicule, et pourvu d'un dispositif capteur (18) installé à l'intérieur de l'habitacle passagers (12), le dispositif capteur (18) étant configuré pour la détection simultanée et/ou séquentielle d'au moins une pluralité de passagers (14) de véhicule à l'intérieur de l'habitacle passagers (12), le dispositif capteur (18) étant agencé mobile,

le véhicule comprenant en outre un dispositif de commande (22) pour au moins une fonction de véhicule, ledit dispositif de commande (22) étant configuré pour déclencher au moins une fonction de véhicule sur la base d'un signal de capteur ou d'une pluralité de signaux de capteur fournis par le dispositif capteur (18) et le dispositif de commande (22) étant configuré pour déclencher des fonctions individuelles et/ou automatiques pour au moins un ou une pluralité de passagers (14) à l'intérieur de l'habitacle passagers (12)

et au moins une cible d'étalonnage (24) étant installée à l'intérieur de l'habitacle passagers (12) à distance du dispositif capteur (18), le dispositif capteur (18) étant configuré pour la détection de la cible d'étalonnage (24) et pour réaliser un étalonnage sur la base d'au moins un facteur d'étalonnage.

2. Véhicule (10) selon la revendication 1, dans lequel le dispositif capteur (18) comprend au moins une unité de capteur optique pour la détection de longueurs d'onde dans le spectre visible et/ou proche infrarouge et/ou au moins une unité de capteur de température pour la détection de longueurs d'onde dans le spectre infrarouge à ondes longues.

3. Véhicule (10) selon la revendication 1 ou 2, dans lequel le dispositif capteur (18) est configuré pour la détection de passagers (14) de véhicule sur la totalité ou au moins une majorité des places d'accueil de passagers à l'intérieur de l'habitacle passagers (12).

4. Véhicule (10) selon l'une des revendications précédentes, dans lequel l'au moins une cible d'étalonnage (24) comprend une surface d'étalonnage (26, 28), dans lequel une pluralité de cibles d'étalonnage (24) sont installées à l'intérieur de l'habitacle (12), et dans lequel le dispositif capteur (18) est configuré pour la détection d'une pluralité de cibles d'étalonnage (24) et pour réaliser un étalonnage sur la base de facteurs d'étalonnage se rapportant à une pluralité de cibles d'étalonnage (24).

5. Véhicule (10) selon la revendication 4, dans lequel un facteur d'étalonnage est une position et/ou une orientation de la cible d'étalonnage (24) à l'intérieur de l'habitacle (12), et/ou dans lequel un facteur d'étalonnage est une température de la cible d'étalonnage (24) .

6. Véhicule (10) selon l'une des revendications 4 ou 5, dans lequel le dispositif capteur (18) est configuré pour la détection de la cible d'étalonnage (24) par forme et/ou différence de température.

7. Véhicule (10) selon l'une des revendications 4 à 6, dans lequel le dispositif capteur (18) est configuré pour déterminer une charge solaire et/ou un rayonnement incident sur la base d'au moins une propriété de surface d'une surface d'étalonnage (26, 28).

8. Véhicule (10) selon l'une des revendications 4 à 7, dans lequel la cible d'étalonnage (24) comprend au moins un dispositif de mesure de température.

9. Véhicule (10) selon l'une des revendications 4 à 8, dans lequel la cible d'étalonnage (24) comprend un dispositif de chauffage et/ou de refroidissement pour le réglage et/ou la régulation de la température de la cible d'étalonnage (24).

10. Véhicule (10) selon l'une des revendications 4 à 9, dans lequel au moins une cible d'étalonnage (24) est un système à température régulée.

11. Système d'étalonnage installé dans un véhicule (10), le système d'étalonnage comprenant un dispositif capteur (18) et au moins une cible d'étalonnage (24),

le véhicule (10) comprenant un habitable passagers (12) destiné à accueillir une pluralité de passagers (14) de véhicule, et un dispositif de commande (22) pour au moins une fonction de véhicule, ledit dispositif de commande (22) étant configuré pour déclencher au moins une fonction de véhicule sur la base d'un signal de capteur ou d'une pluralité de signaux de capteur fournis par le dispositif capteur (18) du système d'étalonnage, et le dispositif de commande (22) étant configuré pour déclencher des fonctions individuelles et/ou automatiques pour au moins un ou une pluralité de passagers (14) à l'intérieur de l'habitacle passagers (12),

le dispositif capteur (18) étant installé agencé mobile à l'intérieur de l'habitacle passagers (12) du véhicule (10), le dispositif capteur (18) étant configuré pour la détection simultanée et/ou séquentielle d'au moins une pluralité de passagers (14) de véhicule à l'intérieur de l'habitacle passagers (12)

et l'au moins une cible d'étalonnage (24) étant installée à l'intérieur de l'habitacle passagers (12) à distance du dispositif capteur (18), le dispositif capteur (18) étant configuré pour la détection de la cible d'étalonnage (24) et pour réaliser un étalonnage sur la base d'au moins un facteur d'étalonnage.

12. Procédé de commande de fonctions d'un véhicule (10) selon l'une des revendications 1 à 10, le procédé comprenant les étapes suivantes :

- détection simultanée et/ou séquentielle d'une pluralité de passagers (14) de véhicule à l'intérieur d'un habitacle passagers (12),
- déclenchement de fonctions de véhicule individualisées pour chacun des passagers (14) à l'intérieur de l'habitacle passagers (12) sur la base de résultats de détection,
- détection d'au moins une cible d'étalonnage (24) par le dispositif capteur (18), la cible d'étalonnage (24) et le dispositif capteur (18) étant installés à l'intérieur de l'habitacle passagers (12) à distance l'un de l'autre, le dispositif capteur (18) étant agencé mobile,
- étalonnage du dispositif capteur (18) sur la base d'au moins un facteur d'étalonnage se rapportant à la cible d'étalonnage (24) détectée.

Fig. 1

Fig. 2

**Fig. 3**

Fig. 4

**Fig. 5a**

**Fig. 5b**

EP 3 718 797 B1

Fig. 6

Fig. 7

**Fig. 8**

Forehead Visible

24c  24b  24a

10

14e  14d  14c  14b  14a

16e  16d  16c  16b  16a

30c

18  22

EP 3 718 797 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 102017200909 A1
- EP 3205547 A1
- DE 102017105871 A1
- DE 102011077993 A1
- US 2018251007 A1
- US 2019061619 A1
- US 2006208874 A1
- DE 102014224489 A1
- US 5994701 A
- US 2013314536 A1
- JP 2004352108 A
- KR 20160128489 A
- US 2007033999 A1
- DE 102015004308